(19) Europäisches Patentamt
European Patent Office
Office européen des brevets

(11) Publication number: 0 377 987 A2

(12) # EUROPEAN PATENT APPLICATION

(21) Application number: 89313373.6

(51) Int. Cl.5: C07D 501/06, C07F 9/09

(22) Date of filing: 20.12.89

The title of the invention has been amended (Guidelines for Examination in the EPO, A-III, 7.3).

(30) Priority: 27.12.88 US 290680

(43) Date of publication of application: 18.07.90 Bulletin 90/29

(84) Designated Contracting States: AT BE CH DE ES FR GB GR IT LI NL SE

(71) Applicant: ELI LILLY AND COMPANY
Lilly Corporate Center
Indianapolis Indiana 46285(US)

(72) Inventor: McShane, Lawrence Joseph
Rural Route 13 Box 232
West Terre Haute Indiana 47885(US)
Inventor: Wirth, David Dale
1219 Lockwood Drive
Lafayette Indiana 47905(US)

(74) Representative: Tapping, Kenneth George et al
Erl Wood Manor
Windlesham Surrey, GU20 6PH(GB)

(54) **Acylation process for obtaining cephalosporin derivatives.**

(57) 7β-[2-(2-Tritylaminothiazol-4-yl )-2-(t-butyloxycarbonylprop-2-yl )oxyiminoacetamido]-3-pyridiniummethyl-3-cephem-4-carboxylate 1 is obtained via a process comprising N-acylation of 7β-amino-3-pyridiniummethyl-3-cephem-4-carboxylate 1A with a mixed anhydride of 2-(2-tritylaminothiazol-4-yl)-2-(t-butyloxycarbonylprop-2-yl)-oxyiminoacetic acid formed with a sulfonic acid halide or phosphoric acid halide, preferably carried out with silylated 1A. The process provides substantial yields of 1 of high purity.

EP 0 377 987 A2

## ACYLATION PROCESS

This invention relates to a chemical acylation process. In particular it relates to an acylation process for the preparation of the antibiotic, ceftazidime.

The broad spectrum semi-synthetic antibiotic ceftazidime is described in U.S. Patent No. 4,258,041. The antibiotic can be prepared by the N-acylation of 7-amino-3-pyridiniummethyl-3-cephem-4-carboxylate with an amino-protected and carboxy-protected derivative of 2- (2-aminothiazol-4-yl)-2-(2-carboxyprop-2-yl) oxyiminoacetic acid. One such derivative is the acid chloride with acylation being carried out in the presence of an acid binding agent. Following acylation the amino and carboxy protecting groups are removed to provide the antibiotic.

Because of the high cost of the 2-aminothiazole oximino acetic acid and the 7-amino-3-pyridiniummethyl-3-cephem-4-carboxylate nucleus improved methods for coupling the two intermediates in higher yield would be highly desirable.

The invention provides a process for the N-acylation of 7-amino-3-pyridiniummethyl-3-cephem- 4-carboxylate (nucleus) with 2-(2-tritylaminothiazol-4-yl) -2-(2-t-butyloxycarbonylprop-2-yl)oxyiminoacetic acid to provide the doubly-blocked ceftazidime which comprises mixing a solution or suspension of a mixed anhydride of the above named iminoacetic acid wherein the mixed anhydride is formed with an alkyl or arylsulfonic acid, e.g., methanesulfonic acid, or a phosphoric acid, e.g., diphenylphosphoric acid; with a solution or suspension of the 7-amino-3-pyridiniummethyl-3-cephem-4-carboxylate in an inert organic solvent in the presence of a tertiary amine e.g., triethylamine or with a solution of silylated 7-amino nucleus compound. The process is carried out at a temperature between about -15 °C and about 20 °C to provide the doubly-blocked ceftazidime in high purity. Preferably the product is isolated as the crystalline bis-DMAC solvate.

According to the process of this invention the amino-protected and carboxy-protected derivative of ceftazidime represented by the formula 1

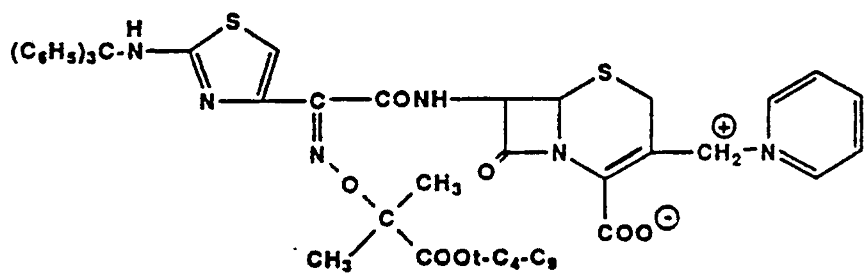

1

is prepared by the N-acylation of the 7-amino-3-pyridiniummethyl nucleus represented by the formula 1A

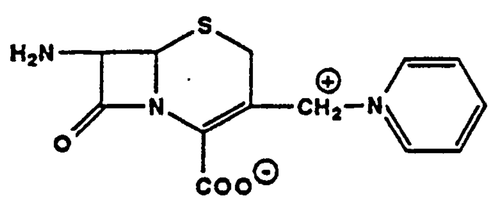

1A

with a mixed anhydride represented by the formula 1B

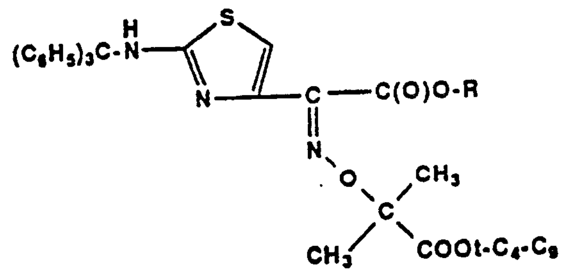

1B

wherein R is an alkyl or aryl sulfonyl group $-SO_2R_1$ wherein $R_1$ is $C_1$-$C_4$ alkyl, phenyl, methylphenyl, halophenyl or naphthyl; or $R_2$ is a phosphate ester group $-P(O)(OR_2)_2$, wherein $R_2$ is $C_1$-$C_4$ alkyl, phenyl, halophenyl or methylphenyl.

The process is carried out in an inert organic solvent at a temperature between -15 °C and about 20 °C preferably at about -10 °C to about 10 °C.

To carry out the process the sulfonic acid anhydride or phosphoric acid anhydride (1B) is prepared as a slurry or solution in an inert organic solvent and the 7-amino-3-pyridiniummethyl-3-cephem-4-carboxylate nucleus (ceftazidime nucleus) 1A, and a tertiary amine such as a trialkylamine e.g. triethylamine or tertiary aromatic amine e.g., N,N-diethylaniline are mixed with the anhydride. A catatylic amount of dimethylaminopyridine (DMAP) enhances the rate of the N-acylation and is preferably added to the mixture. The ceftazidime nucleus can be in the form of the stable dihydrochloride salt or dihydrobromide salt, preferably the crystalline salt.

Organic solvents which can be used in the process are those solvents in which the anhydride and the nucleus are at least partially soluble. Preferred solvents are the amide solvents such as dimethylformamide and dimethylacetamide. Dimethylacetamide is preferred since it forms a less soluble crystalline bis solvate with the acylation product, doubly-blocked ceftazidime (1).

Alternatively, the ceftazidime nucleus can be silylated in an inert organic solvent to form a solution of the soluble silylated derivative and the solution mixed with a solution of the anhydride to effect acylation. For example, the ceftazidime nucleus 1A as the dihydrochloride dihydrate can be silylated in a chlorinated hydrocarbon solvent such as methylene chloride, chloroform or trichloroethane with a silylating reagent such as, for example, bis-(trimethylsilyl)urea (BSU), mono- or bis-trimethylsilylacetamide or N-trimethylsilyloxazolidone to form a solution of the silylated nucleus. Other solvents suitable for use in this aspect of the process include toluene, ethyl acetate, acetone, tetrahydrofuran, or acetonitrile.

The nucleus (1A) and the mixed anhydride (1B) are used in equimolar proportions and preferably an excess amount of the mixed anhydride 1B is used. The mixed anhydride 1B can be added to the solution or suspension of the nucleus 1A or the solution or suspension of the nucleus can be added to a solution of the mixed anhydride.

The process of this invention proceeds rapidly and with laboratory scale reactions is generally complete in about an hour or less. The process affords doubly-blocked ceftazidime of high quality, as shown by high performance liquid chromatographic (HPLC) assay, and generally in high yields.

A preferred process of the invention comprises the use of silylated ceftazidime nucleus 1A. A preferred mixed anhydride is represented by formula 1B wherein R is the group $-SO_2R_1$, in particular wherein $R_1$ is methyl. In an example of the preferred process 2-(2-tritylaminothiazol-4-yl )-2-(2-t-butyloxycarbonylprop-2-yl) oxyiminoacetic acid is converted in DMAC to the mixed anhydride formed with methanesulfonic acid as described above and is mixed at a temperature of about 0 °C with a solution of silylated nucleus 1A to provide doubly-blocked ceftazidime 1.

The doubly-blocked ceftazidime product 1 is readily isolated from the reaction mixture as the crystalline bis-DMAC solvate by following conventional procedures. In the preferred embodiment of the process wherein the silylated nucleus is used the reaction mixture is diluted with water following acylation and the pH of the aqueous mixture adjusted to near neutrality with base. Thereafter the organic phase containing the product is separated and is treated with DMAC to form the solvate. Alternatively the product can be isolated in the non-solvated form by extraction from the neutralized reaction mixture.

The mixed anhydrides represented by the formula 1B are readily prepared for use in the process as follows. The 2-(2-tritylaminothiazol-4-yl)-2-(2-t-butyloxycarbonylprop-2- 1)oxyiminoacetic acid is suspended in a solvent such as DMF, toluene, ether or, preferably, DMAC or dichloromethane and the suspension treated with an alkylsulfonyl chloride, arylsulfonyl chloride or phosphoric acid chloride and a tertiary alkyl amine e.g. triethylamine. The preparation is best carried out in the cold at a temperature between about -15 °C and 10 °C. The solution or suspension of the mixed anhydride can be conveniently used as such in

the process and the anhydride need not be isolated.

Examples of sulfonyl chlorides which are used to prepare 1B are methanesulfonyl chloride, ethanesulfonyl chloride, n-butanesulfonyl chloride, benzenesulfonyl chloride, p-chlorobenzenesulfonyl chloride, p-toluenesulfonyl chloride, and the like. Examples of diesters of phosphoric acid chloride, $Cl-P(O)(OR_2)_2$, are diethylphosphoric acid chloride, dimethylphosphoric acid chloride, diphenylphosphoric acid chloride, and di-(4-methylphenyl)phosphoric acid chloride. The corresponding sulfonylbromides and phosphoric acid bromides also can be used in the preparation of the mixed anhydrides. A preferred anhydride of this invention is represented by the formula 1B wherein R is $-SO_2R_1$ and $R_1$ is methyl. Another preferred mixed anhydride 1B is represented when $R_2$ is ethyl.

The doubly-blocked ceftazidime provided by the process is converted to the antibiotic ceftazidime by removal of the trityl protecting group and the t-butyl protecting groups with a mixture of hydrobromic acid and formic acid by known procedures.

The following Examples further illustrate and describe the process of this invention and are not to be construed as limiting thereof.

## Example 1

Acylation with mixed anhydride of methanesulfonic acid and 2-(2-tritylaminothiazol-4-yl)-2-(t-butyloxycarbonylprop-2-yl)oxyiminoacetic acid.

A slurry of 2-(2-tritylaminothiazol-4-yl)-2-(t-butyloxycarbonylprop-2-yl)oxyiminoacetic acid (8.0 g, 14 mmole) in 44 ml of dimethylacetamide (DMAC) was cooled to about 2 °C and methanesulfonyl chloride (1.08 ml, 14 mmole) was added followed by the dropwise addition over 5 min. of triethylamine (3.9 ml, 28 mmole). The slurry was stirred 2 h at about 1 °C and then 3.9 ml of additional triethylamine were added in one portion followed by the addition of 5.6 g (14 mmole) of 7-amino-3-pyridiniummethyl-3-cephem-4-carboxylate dihydrochloride dihydrate and a catalytic amount of dimethylaminopyridine. The reaction mixture was stirred in the cold for 2 h and was diluted with 60 ml of methylene chloride followed by 100 ml of water. The organic layer was separated and treated with 100 ml of ethyl acetate, 50 ml of diethyl ether and 50 ml of DMAC and was seeded with crystalline doubly-blocked ceftazidime. Crystallization occurred and after 1 h at room temperature the slurry was refrigerated overnight. The cold slurry was filtered and the product was washed with 25 ml of 1:1 DMAC:diethyl ether and with 25 ml of diethyl ether. The washed product was air dried overnight at 30 °C to provide 6.62 g (52.4% yield) of doubly-blocked ceftazidime as the bis-DMAC solvate (93.7% via HPLC assay).

## Example 2

Acylation with mixed anhydride of ethanesulfonic acid.

The acylation described by Example 1 was carried out with 1B ($R_1 = C_2H_5$) formed with 16.9 mmole (10.1 g) of the oximinoacetic acid and ethanesulfonyl chloride instead of methanesulfonyl chloride and 5.6 g (14 mmol) of the pyridinium nucleus. There were obtained 6.08 g (49.3% yield) having an HPLC assay of 90.7% of doubly-blocked ceftazidime bis-DMAC solvate.

## Example 3

Acylation with mixed anhydride of p-toluenesulfonic acid

The procedure described by Example 1 was employed in acylating 5.6 g (14 mmole) of the ceftazidime nucleus with the anhydride formed with 8.0 g (14 mmole) of the oximinoacetic acid and 2.7 g (14 mmole) of p-toluenesulfonyl chloride. There were obtained 4.5 g of doubly-blocked ceftazidime bis-DMAC solvate (HPLC assay of 93.2%).

## Example 4

Acylation with mixed anhydride of diphenylphosphoric acid

The procedure described by Example 1 was used in acylating 5.6 g (14 mmole) of the ceftazidime nucleus (1A) with the anhydride formed with 8.0 g (14 mmole) of the oximinoacetic acid and 2.9 ml (14 mmole) of diphenyl phosphoric acid chloride. There were obtained 5.06 g of doubly-blocked ceftazidime bis-DMAC solvate (39.5% yield). HPLC assay - 92.1%.

## Example 5

Acylation of silylated nucleus with the mixed anhydride formed with methanesulfonic acid

A slurry of 8.31 g of the oximinoacetic acid in 50 ml of methlene chloride was cooled to 0° C, 3.6 ml of triethylamine were added and the solution was cooled to -10° C. A solution of 1.7 ml of methanesulfonyl chloride in 5 ml of methylene chloride was added to the cold solution over 2 min and, after addition, the solution was stirred for 5 min at -10° C to -5° C.

In a separate reaction vessel 15.1 g of bis-(trimethylsilyl)urea was slurried in 50 ml of methylene chloride, 3.5 g of 1A were added and the mixture was stirred for 2.5 h at room temperature. The silylated nucleus solution was cooled to 0° C and was mixed all at once with the cold solution of the mixed anhydride prepared as described above. The reaction mixture was stirred in the cold for 45 min, 85 ml of water were added and the pH was adjusted from 2 to 6.5 by the addition of about 5 ml of triethylamine. An emulsion formed and was broken by adding 25 ml of DMAC and heating the mixture to 25° C. The organic layer was separated from the aqueous phase and the latter extracted with 10 ml of methylene chloride. The extract was combined with the organic layer and the whole evaporated to a concentrate weighing 40 g. The concentrate was diluted with 10 ml of DMAC and 10 ml of ethyl acetate, was seeded and then stirred for 45 min to form a crystalline slurry. A mixture of DMAC (15 ml) and diethylether (50 ml) was added dropwise over 1 h and after addition the slurry was stirred for .5 h at room temperature and then in an ice bath for .75 h. The crystalline product was filtered and washed with a mixture of 15 ml of DMAC and 15 ml of ethyl acetate and finally with 30 ml of ethyl acetate. The washed crystals were dried at 30° C in vacuo to yield 9.06 g of doublyblocked ceftazidime DMAC solvate.

## Claims

1. A process or preparing the compound of formula 1

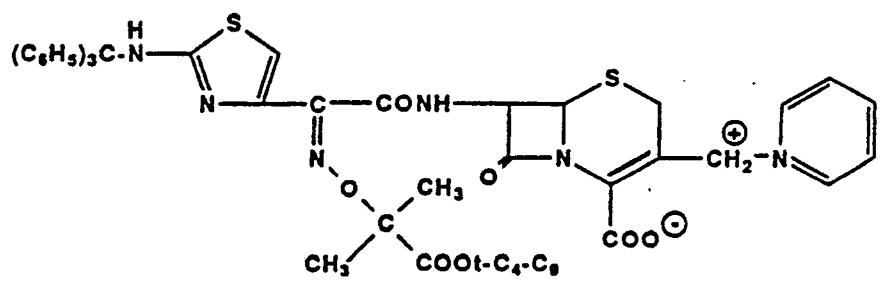

1

which comprises mixing in an inert organic solvent at a temperature between about -15° C and about 20° C a mixed anhydride of formula 1B

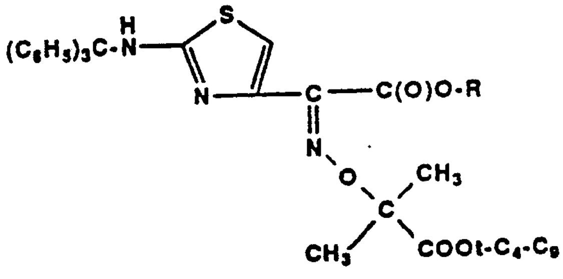

1B

with the amino pyridinium compound of formula 1A

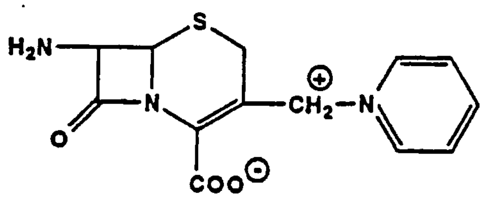

1A

wherein R is a sulfonyl group of the formula $-SO_2R_1$ wherein $R_1$ is $C_1$-$C_4$ alkyl, phenyl, methylphenyl, halophenyl or naphthyl; or R is a phosphate ester group $-P(O)(OR_2)_2$ wherein $R_2$ is $C_1$-$C_4$ alkyl, phenyl, methylphenyl or halophenyl.

2. The process of claim 1 wherein the amino pyridinium nucleus compound is mixed with the mixed anhydride in the presence of a tertiary amine.

3. The process of claim 2 wherein the inert organic solvent is DMF or DMAC.

4. The process of claim 1 wherein the amino-pyridinium nucleus compound is in the form of a trialkylsilylated derivative.

5. The process of claim 4 wherein the silylated derivative is a trimethylsilylated derivative.

6. The process of claim 1 wherein the mixed anhydride R is the sulfonyl group $-SO_2R_1$.

7. The process of claim 6 wherein $R_1$ is methyl or ethyl.

8. The process of claim 1 wherein the mixed anhydride R is the group $-P(O)(OR_2)_2$.